# EUROPEAN PATENT APPLICATION

(11) **EP 4 759 320 A1**
(43) Date of publication of application: **17.06.2026**
(21) Application number: 24854427.2
(22) Date of filing: 12.08.2024
(51) Int. Cl.: A61K 35/545, A61P 27/02

(54) **PHARMACEUTICAL COMPOSITION FOR PREVENTING OR TREATING CORNEAL DISEASES COMPRISING CORNEAL ENDOTHELIAL CELLS INDUCED TO DIFFERENTIATE**

(30) Priority: 11.08.2023 KR 20230105484
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: KIM, Jae Yong, Seoul 04984 (KR); LEE, Hun, Seoul 06503 (KR); CHUNG, Ho Seok, Seoul 06107 (KR)
(74) Representative: AWA Sweden AB
(86) International application number: PCT/KR2024/011949
(87) International publication number: WO 2025/037876

(57) **Abstract**

According to an aspect, when differentiation-induced corneal endothelial cells and stem cells of a composition are administered in combination, the composition is effective for preventing or treating corneal diseases by improving damaged corneal endothelial cells, thereby improving corneal opacity, decreasing corneal thickness, reducing intracorneal inflammation, and alleviating corneal fibrosis.

## Description

### Technical Field

The present disclosure relates to a pharmaceutical composition for preventing or treating a corneal disease, including differentiation-induced corneal endothelial cells.

### Background Art

The cornea is the outermost structure of the eyeball and serves as one of the primary organs for refracting light. The cornea consists of a total of six layers: the corneal epithelium, Bowman's layer, corneal stroma, Dua's layer, Descemet's membrane, and the corneal endothelium.

The corneal endothelium is a monolayer of hexagonal cells located on the posterior surface of the cornea and contains an embedded physiological ion pump. The ion pump embedded in the corneal endothelium maintains corneal transparency by removing excess water from the corneal stroma. If this water regulation function fails, the cornea swells and becomes opaque, which may lead to vision loss.

Meanwhile, since corneal endothelial cells (CECs) have limited proliferative capacity in adults, damaged CECs are repaired through cell expansion and migration rather than mitosis. Consequently, the cornea swells to several times its normal thickness, becomes opaque, and loses its function. Such damage is permanently maintained.

Accordingly, the present inventors have developed a composition capable of preventing or treating corneal diseases by injecting stem cell-derived corneal endothelial cells and stem cells to improve damaged corneal endothelial cells. This composition restores corneal transparency by assisting in the replacement or repair of damaged corneal endothelial cells and is effective in reducing corneal edema by restoring the water regulation function of the corneal endothelium.

### Disclosure of Invention

### Technical Problem

One aspect of the present disclosure is to provide a pharmaceutical composition for preventing or treating a corneal disease, the composition including differentiation-induced corneal endothelial cells (CECs) and stem cells.

Another aspect is to provide a formulation for combined administration for preventing or treating a corneal disease, including a first agent including differentiation-induced corneal endothelial cells and a second agent including stem cells.

Another aspect is to provide a cell therapy product for preventing or treating a corneal disease, including differentiation-induced corneal endothelial cells and stem cells.

Another aspect is to provide a use of a composition including differentiation-induced corneal endothelial cells and stem cells for preventing or treating a corneal disease.

Another aspect is to provide a method of preventing or treating a corneal disease, the method including administering a composition including an effective amount of differentiation-induced corneal endothelial cells and stem cells to a subject in need thereof.

Another aspect is to provide a use of a composition including differentiation-induced corneal endothelial cells and stem cells for the manufacture of a medicament for preventing or treating a corneal disease.

### Solution to Problem

One aspect of the present disclosure provides a pharmaceutical composition for preventing or treating a corneal disease, the composition including differentiation-induced corneal endothelial cells (CECs) and stem cells.

The term "corneal endothelial cell (CEC)" as used herein may refer to a cell constituting the corneal endothelium. Specifically, the CECs are cells in a single layer located at the innermost part of the cornea and play an important role in maintaining the transparency and moisture balance of the eye. To maintain the transparency of the cornea, the CECs use an ion pump, particularly Na⁺/K⁺ ATPase, to move water from the stroma to the aqueous humor in order to regulate and prevent excessive influx of moisture into the cornea.

In an embodiment, the differentiation-induced corneal endothelial cells may be those induced to differentiate from embryonic stem cells (ESCs), adult stem cells, or human induced pluripotent stem cells (iPSCs). For example, the differentiation-induced corneal endothelial cells may be induced to differentiate from mesenchymal stem cells (MSCs), and may be induced to differentiate from any one selected from the group consisting of stem cells derived from bone marrow, blood, umbilical cord blood, umbilical cord, adipose tissue, liver, skin, gastrointestinal tract, placenta, and uterus, but are not limited thereto.

The term "stem cell" as used herein may refer to a totipotent cell capable of differentiating into all types of cells or a pluripotent cell capable of differentiating into several types of cells, and the stem cell is an undifferentiated cell that can be differentiated into cells of a specific tissue.

In an embodiment, the stem cells may be embryonic stem cells (ESCs), adult stem cells, or induced pluripotent stem cells (iPSCs).

The embryonic stem cells may be those cultured *in vitro* after extracting an inner cell mass from a blastocyst-stage embryo just before a fertilized egg is implanted into a maternal uterus, and the adult stem cells may be undifferentiated cells that exist in an extremely small amount in each tissue of the body and replace dead cells or damaged tissues. The induced pluripotent stem cells (iPSCs) may be cells in which pluripotency is induced similar to embryonic stem cells by injecting cell differentiation-related genes into differentiated somatic cells to revert them to a cell stage prior to differentiation.

In an embodiment, the adult stem cells may be mesenchymal stem cells (MSCs), but are not limited thereto.

In an embodiment, the adult stem cells may be selected from the group consisting of stem cells derived from bone marrow, blood, umbilical cord blood, umbilical cord, adipose tissue, liver, skin, gastrointestinal tract, placenta, and uterus, but are not limited thereto.

The term "differentiation" as used herein may refer to a phenomenon in which structures or functions become specialized while cells divide, proliferate, and grow, i.e., a process in which the morphology or function of biological cells, tissues, and the like change to perform tasks assigned to each.

The term "induction of differentiation from stem cells into corneal endothelial cells" as used herein may include not only cases in which complete differentiation from stem cells including iPSCs and the like into corneal endothelial cells is induced, but also cases in which differentiation into cells expressing corneal endothelial cell-specific markers is induced.

The term "culture medium for inducing differentiation from stem cells into corneal endothelial cells" as used herein may refer to any medium that can induce differentiation of stem cells into corneal endothelial cells by decreasing characteristics as pluripotent cells and increasing corneal endothelial cell-specific characteristics during the process of inducing differentiation of stem cells into corneal endothelial cells. For example, the culture medium may decrease the expression of pluripotent stem cell-specific markers or increase the expression of corneal endothelial cell-specific markers.

In an embodiment, the pharmaceutical composition for preventing or treating a corneal disease may be characterized by satisfying at least one of the following characteristics:
- Improving corneal opacity;
- Decreasing corneal thickness;
- Improving corneal pump function and intercellular adhesion;
- Decreasing the expression of inflammatory cytokines;
- Decreasing intracorneal apoptosis;
- Decreasing corneal fibrosis; and
- Mitigating allograft rejection reactions.

In an embodiment, the composition for preventing or treating a corneal disease may include the differentiation-induced corneal endothelial cells and the stem cells in a ratio of 0.5 to 2 : 0.5 to 2. For example, the ratio of the differentiation-induced corneal endothelial cells and the stem cells may be 0.5 : 0.5, 0.5 : 0.6, 0.5 : 0.7, 0.5 : 0.8, 0.5 : 0.9, 0.5 : 1.0, 0.5 : 1.1, 0.5 : 1.2, 0.5 : 1.3, 0.5 : 1.4, 0.5 : 1.5, 0.5 : 1.6, 0.5 : 1.7, 0.5 : 1.8, 0.5 : 1.9, 0.5 : 2.0, 0.6 : 0.5, 0.6 : 0.6, 0.6 : 0.7, 0.6 : 0.8, 0.6 : 0.9, 0.6 : 1.0, 0.6 : 1.1, 0.6 : 1.2, 0.6 : 1.3, 0.6 : 1.4, 0.6 : 1.5, 0.6 : 1.6, 0.6 : 1.7, 0.6 : 1.8, 0.6 : 1.9, 0.6 : 2.0, 0.7 : 0.5, 0.7 : 0.6, 0.7 : 0.7, 0.7 : 0.8, 0.7 : 0.9, 0.7 : 1.0, 0.7 : 1.1, 0.7 : 1.2, 0.7 : 1.3, 0.7 : 1.4, 0.7 : 1.5, 0.7 : 1.6, 0.7 : 1.7, 0.7 : 1.8, 0.7 : 1.9, 0.7 : 2.0, 0.8 : 0.5, 0.8 : 0.6, 0.8 : 0.7, 0.8 : 0.8, 0.8 : 0.9, 0.8 : 1.0, 0.8 : 1.1, 0.8 : 1.2, 0.8 : 1.3, 0.8 : 1.4, 0.8 : 1.5, 0.8 : 1.6, 0.8 : 1.7, 0.8 : 1.8, 0.8 : 1.9, 0.8 : 2.0, 0.9 : 0.5, 0.9 : 0.6, 0.9 : 0.7, 0.9 : 0.8, 0.9 : 0.9, 0.9 : 1.0, 0.9 : 1.1, 0.9 : 1.2, 0.9 : 1.3, 0.9 : 1.4, 0.9 : 1.5, 0.9 : 1.6, 0.9 : 1.7, 0.9 : 1.8, 0.9 : 1.9, 0.9 : 2.0, 1.0 : 0.5, 1.0 : 0.6, 1.0 : 0.7, 1.0 : 0.8, 1.0 : 0.9, 1.0 : 1.0, 1.0 : 1.1, 1.0 : 1.2, 1.0 : 1.3, 1.0 : 1.4, 1.0 : 1.5, 1.0 : 1.6, 1.0 : 1.7, 1.0 : 1.8, 1.0 : 1.9, 1.0 : 2.0, 1.1 : 0.5, 1.1 : 0.6, 1.1 : 0.7, 1.1 : 0.8, 1.1 : 0.9, 1.1 : 1.0, 1.1 : 1.1, 1.1 : 1.2, 1.1 : 1.3, 1.1 : 1.4, 1.1 : 1.5, 1.1 : 1.6, 1.1 : 1.7, 1.1 : 1.8, 1.1 : 1.9, 1.1 : 2.0, 1.2 : 0.5, 1.2 : 0.6, 1.2 : 0.7, 1.2 : 0.8, 1.2 : 0.9, 1.2 : 1.0, 1.2 : 1.1, 1.2 : 1.2, 1.2 : 1.3, 1.2 : 1.4, 1.2 : 1.5, 1.2 : 1.6, 1.2 : 1.7, 1.2 : 1.8, 1.2 : 1.9, 1.2 : 2.0, 1.3 : 0.5, 1.3 : 0.6, 1.3 : 0.7, 1.3 : 0.8, 1.3 : 0.9, 1.3 : 1.0, 1.3 : 1.1, 1.3 : 1.2, 1.3 : 1.3, 1.3 : 1.4, 1.3 : 1.5, 1.3 : 1.6, 1.3 : 1.7, 1.3 : 1.8, 1.3 : 1.9, 1.3 : 2.0, 1.4 : 0.5, 1.4 : 0.6, 1.4 : 0.7, 1.4 : 0.8, 1.4 : 0.9, 1.4 : 1.0, 1.4 : 1.1, 1.4 : 1.2, 1.4 : 1.3, 1.4 : 1.4, 1.4 : 1.5, 1.4 : 1.6, 1.4 : 1.7, 1.4 : 1.8, 1.4 : 1.9, 1.4 : 2.0, 1.5 : 0.5, 1.5 : 0.6, 1.5 : 0.7, 1.5 : 0.8, 1.5 : 0.9, 1.5 : 1.0, 1.5 : 1.1, 1.5 : 1.2, 1.5 : 1.3, 1.5 : 1.4, 1.5 : 1.5, 1.5 : 1.6, 1.5 : 1.7, 1.5 : 1.8, 1.5 : 1.9, 1.5 : 2.0, 1.6 : 0.5, 1.6 : 0.6, 1.6 : 0.7, 1.6 : 0.8, 1.6 : 0.9, 1.6 : 1.0, 1.6 : 1.1, 1.6 : 1.2, 1.6 : 1.3, 1.6 : 1.4, 1.6 : 1.5, 1.6 : 1.6, 1.6 : 1.7, 1.6 : 1.8, 1.6 : 1.9, 1.6 : 2.0, 1.7 : 0.5, 1.7 : 0.6, 1.7 : 0.7, 1.7 : 0.8, 1.7 0.9, 1.7 1.0, 1.7 1. 1, 1.7 1.2, 1.7 1.3, 1.7 1.4, 1.7 : 1.5, 1.7 : 1.6, 1.7 : 1.7, 1.7 : 1.8, 1.7 : 1.9, 1.7 : 2.0, 1.8 : 0.5, 1.8 : 0.6, 1.8 : 0.7, 1.8 : 0.8, 1.8 : 0.9, 1.8 : 1.0, 1.8 : 1.1, 1.8 : 1.2, 1.8 : 1.3, 1.8 : 1.4, 1.8 : 1.5, 1.8 : 1.6, 1.8 : 1.7, 1.8 : 1.8, 1.8 : 1.9, 1.8 : 2.0, 1.9 : 0.5, 1.9 : 0.6, 1.9 : 0.7, 1.9 : 0.8, 1.9 : 0.9, 1.9 : 1.0, 1.9 : 1.1, 1.9 : 1.2, 1.9 : 1.3, 1.9 : 1.4, 1.9 : 1.5, 1.9 : 1.6, 1.9 : 1.7, 1.9 : 1.8, 1.9 : 1.9, 1.9 : 2.0, 2.0 : 0.5, 2.0 : 0.6, 2.0 : 0.7, 2.0 : 0.8, 2.0 : 0.9, 2.0 : 1.0, 2.0 : 1.1, 2.0 : 1.2, 2.0 : 1.3, 2.0 : 1.4, 2.0 : 1.5, 2.0 : 1.6, 2.0 : 1.7, 2.0 : 1.8, 2.0 : 1.9, or 2.0 : 2.0, but is not limited thereto.

In an embodiment, the composition for preventing or treating a corneal disease may include the differentiation-induced corneal endothelial cells in an amount of 1×10⁴ cells to 1×10⁷ cells. For example, the composition for preventing or treating a corneal disease may include the induced corneal endothelial cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5×10⁶ cells, 6×10⁶ cells, 7×10⁶ cells, 8×10⁶ cells, 9×10⁶ cells, or 1×10⁷ cells, but is not limited thereto.

In an embodiment, the composition for preventing or treating a corneal disease may include the stem cells in an amount of 1×10⁴ cells to 1×10⁷ cells. For example, the composition for preventing or treating a corneal disease may include the stem cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5×10⁶ cells, 6×10⁶ cells, 7×10⁶ cells, 8×10⁶ cells, 9×10⁶ cells, or 1×10⁷ cells, but is not limited thereto.

The term "corneal disease" as used herein refers to any disease or damage in which corneal cells are damaged such that the transparency of the cornea cannot be maintained, and specifically, the corneal disease may be at least one selected from the group consisting of corneal endothelial decompensation, Fuchs' dystrophy, corneal endothelial cell dystrophy, corneal edema, posterior polymorphous corneal dystrophy (PPCD), and corneal transplant rejection, but is not limited thereto.

The term "treat" as used herein may refer to healing a corneal disease or the like in a shortened time compared to natural healing. The treatment may include improvement and/or alleviation of the corneal disease. In addition, the treatment may refer to healing and/or recovery of symptoms caused by the corneal disease.

The term "prevention" as used herein refers to a method of partially or completely delaying or preventing the onset or recurrence of a disease, disorder, or incidental symptoms thereof, preventing the acquisition or re-acquisition of a disease or disorder, or reducing the risk of acquisition of a disease or disorder. For example, the prevention refers to all acts of suppressing or delaying the occurrence of a corneal disease by administering the composition according to the present disclosure.

For administration, the pharmaceutical composition may include a pharmaceutically acceptable carrier, an excipient, or a diluent in addition to the active ingredients described above.

The term "pharmaceutically acceptable carrier" as used in the present disclosure may refer to a carrier or a diluent that does not irritate an organism and does not inhibit the biological activity and properties of an injected compound. The types of the carrier usable in the present disclosure are not particularly limited, and any carrier conventionally used in the art and pharmaceutically acceptable may be used. Non-limiting examples of the carrier include saline, sterile water, Ringer's solution, buffered saline, albumin injection solution, dextrose solution, maltodextrin solution, glycerol, and the like. Such carriers may be used alone or in a combination of two or more. The carrier may include a non-naturally occurring carrier. In addition, other conventional additives such as an antioxidant, a buffer, and/or a bacteriostat may be added and used if necessary, and the composition may be formulated into an injectable formulation such as an aqueous solution, a suspension, or an emulsion by additionally adding a diluent, a dispersant, a surfactant, a binder, a lubricant, or the like.

A suitable dosage of the pharmaceutical composition of the present disclosure varies depending on a patient's condition and weight, degree of disease, drug form, and time, but may be appropriately selected by those skilled in the art.

The term "pharmaceutically effective amount" as used herein refers to an amount sufficient to treat a disease with a reasonable benefit/risk ratio applicable to medical treatment, and generally, 1 to 2 million cells per eye can be administered once to several times a day in divided doses. However, for the purposes of the present disclosure, the specific therapeutically effective amount for a specific patient is preferably determined depending on various factors including the type and degree of response to be achieved, the specific composition (including whether other agents are used in some cases), the patient's age, weight, general health condition, sex, and diet, the time of administration, the route of administration, the secretion rate of the composition, the duration of treatment, and drugs used in combination or simultaneously with the specific composition, as well as similar factors well known in the medical field.

The pharmaceutical composition of the present disclosure may be administered as an individual therapeutic agent or in combination with other therapeutic agents, and may be administered sequentially or simultaneously with conventional therapeutic agents. The pharmaceutical composition may be administered in a single dose or multiple doses. It is important to administer a dose capable of achieving the maximum effect with a minimum amount without causing side effects in consideration of all the above factors, and such a dose may be readily determined by those skilled in the art.

The term "administration" as used in the present disclosure refers to introducing the pharmaceutical composition of the present disclosure to a patient by any appropriate method, and since the pharmaceutical composition including differentiation-induced corneal endothelial cells (CECs) and stem cells is effective in preventing or treating a corneal disease or a conjunctival disease, the composition may be administered via the eye.

Another aspect provides a formulation for combined administration for preventing or treating a corneal disease, including differentiation-induced corneal endothelial cells (CECs) as a first agent and stem cells as a second agent.

In an embodiment, the first agent and the second agent may be for direct administration to the eye.

In an embodiment, the first agent and the second agent may be administered in combination simultaneously, sequentially, or in reverse order.

The terms "combination," "combined treatment," or "in combination" as used herein refer to any form of simultaneous or concurrent treatment using at least two separate therapeutic agents. Components of the combination therapy may be administered simultaneously, sequentially, in reverse order, or in any order. The components may be administered in an appropriate manner at different dosages, at different administration frequencies, or through different routes.

The combined administration may mean administering the first agent including differentiation-induced corneal endothelial cells (CECs) and the second agent including stem cells simultaneously, sequentially, or individually, and in any order.

The term "simultaneously administered" as used herein is not especially limited and refers to the components of a combination therapy being administered substantially simultaneously, for example, as a mixture or in an immediately following order.

The term "sequentially administered" as used herein is not especially limited and refers to the components of a combination therapy not being administered simultaneously but being administered one by one or in batches with a specific time interval between administrations. The time interval may be the same or different between respective administrations of the components of the combination therapy, and may be selected, for example, from a range of 2 minutes to 96 hours, 1 day to 7 days, or 1 week, 2 weeks, or 3 weeks. Generally, the time interval between administrations may range from several minutes to several hours, for example, 2 minutes to 72 hours, 30 minutes to 24 hours, or 1 to 12 hours. Additional examples include time intervals in the range of 24 to 96 hours, 12 to 36 hours, 8 to 24 hours, and 6 to 12 hours.

For example, the combined administration may involve administering a first agent including differentiation-induced corneal endothelial cells (CECs) and a second agent including stem cells simultaneously, or administering the first agent including the differentiation-induced corneal endothelial cells and then administering the second agent. The combination therapy according to the present disclosure may be defined as providing a synergistic effect if the efficacy measured, for example, through the degree of response, the rate of response, or the time to disease progression, is therapeutically superior to the efficacy obtainable by administering the individual components of the combination therapy at conventional doses. For example, if efficacy of the combination therapy is therapeutically superior to efficacy obtained by using each of the components alone, the efficacy of the combination therapy is synergistic. In particular, a synergistic effect is considered to exist if the conventional doses of the materials containing differentiation-induced corneal endothelial cells (CECs) and stem cells can be reduced with fewer and/or less problematic side effects than those occurring when using each component at a conventional dose, without adversely affecting at least one of the degree of response, the rate of response, and the time to disease progression, and especially without compromising the duration of response.

The term "administration" as used herein refers to introducing a specific substance to a subject by an appropriate method, and "subject" refers to all living organisms such as rats, mice, and livestock, including humans, that can suffer from a corneal disease. As a specific example, the subject may be a mammal including a human.

The term "formulation for combined administration" as used herein refers to a combination designed to use two or more drugs or therapeutic agents together for the purpose of preventing or treating corneal diseases. For example, the formulation for combined administration may refer to a combination designed to administer a first agent including differentiation-induced corneal endothelial cells (CECs) and a second agent including stem cells simultaneously, sequentially, or individually, and in any order. Specifically, the agent may be in a formulation selected from the group consisting of eye drops, injections, eye ointments, ocular inserts, instillations, gels, suspensions, and emulsions, but is not limited thereto.

In an embodiment, the first agent may include the differentiation-induced corneal endothelial cells (CECs) in an amount of 1×10⁴ cells to 1×10⁷ cells. For example, the first agent may include the induced corneal endothelial cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5×10⁶ cells, 6×10⁶ cells, 7×10⁶ cells, 8×10⁶ cells, 9×10⁶ cells, or 1×10⁷ cells, but is not limited thereto.

In an embodiment, the second agent may include the stem cells in an amount of 1×10⁴ cells to 1×10⁷ cells. For example, the second agent may include the stem cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5×10⁶ cells, 6×10⁶ cells, 7×10⁶ cells, 8×10⁶ cells, 9×10⁶ cells, or 1×10⁷ cells, but is not limited thereto.

Another aspect provides a cell therapy product for preventing or treating a corneal disease, including differentiation-induced corneal endothelial cells (CECs) and stem cells.

The term "cell therapy product" as used herein may refer to a therapeutic modality using living cells to treat a patient's disease or damaged tissue. Specifically, the term may refer to a therapeutic agent that induces tissue regeneration, immunomodulation, or recovery of specific cell functions by administering cells to a patient after culturing or manipulating the cells *in vitro.*

In an embodiment, the cell therapy product for preventing or treating a corneal disease may be a cell therapy product characterized by satisfying at least one of the following features.
- Improving corneal opacity;
- Decreasing corneal thickness;
- Improving corneal pump function and intercellular adhesion;
- Decreasing the expression of inflammatory cytokines;
- Decreasing intracorneal apoptosis;
- Decreasing corneal fibrosis; and
- Mitigating allograft rejection reactions.

In an embodiment, the cell therapy product for preventing or treating a corneal disease may be one in which the stem cells are embryonic stem cells (ESCs), adult stem cells, or human induced pluripotent stem cells (iPSCs).

In an embodiment, the cell therapy product for preventing or treating a corneal disease may be one in which the differentiation-induced corneal endothelial cells are induced to differentiate from embryonic stem cells (ESCs), adult stem cells, or human induced pluripotent stem cells (iPSCs).

In an embodiment, the cell therapy product may include the differentiation-induced corneal endothelial cells in an amount of 1×10⁴ cells to 1×10⁷ cells, but is not limited thereto. For example, the cell therapy product may include the induced corneal endothelial cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5x10⁶ cells, 6×10⁶ cells, 7×10⁶ cells, 8×10⁶ cells, 9×10⁶ cells, or 1×10⁷ cells, but is not limited thereto.

In an embodiment, the cell therapy product may include the stem cells in an amount of 1×10⁴ cells to 1×10⁷ cells, but is not limited thereto. For example, the cell therapy product may include the stem cells in an amount of 1×10⁴ cells, 2×10⁴ cells, 3×10⁴ cells, 4×10⁴ cells, 5×10⁴ cells, 6×10⁴ cells, 7×10⁴ cells, 8×10⁴ cells, 9×10⁴ cells, 1×10⁵ cells, 2×10⁵ cells, 3×10⁵ cells, 4×10⁵ cells, 5×10⁵ cells, 6×10⁵ cells, 7×10⁵ cells, 8×10⁵ cells, 9×10⁵ cells, 1×10⁶ cells, 2×10⁶ cells, 3×10⁶ cells, 4×10⁶ cells, 5×10⁶ cells, 6×10⁶ cells, 7×10⁶ cells, 8×10⁶ cells, 9×10⁶ cells, or 1×10⁷ cells, but is not limited thereto.

In an embodiment, the cell therapy product may include the differentiation-induced corneal endothelial cells and the stem cells in a ratio of 0.5 to 2 : 0.5 to 2, and for example, may include them in a ratio of 1.0 : 0.5, 1.0 : 0.6, 1.0 : 0.7, 1.0 : 0.8, 1.0 : 0.9, 1.0 : 1.0, 1.0 : 1.1, 1.0 : 1.2, 1.0 : 1.3, 1.0 : 1.4, or 1.0 : 1.5. More specifically, the cell therapy product may include the differentiation-induced corneal endothelial cells and the stem cells in a ratio of 0.5 : 0.5, 0.5 : 0.6, 0.5 : 0.7, 0.5 : 0.8, 0.5 : 0.9, 0.5 : 1.0, 0.5 : 1.1, 0.5 : 1.2, 0.5 : 1.3, 0.5 : 1.4, 0.5 : 1.5, 0.5 : 1.6, 0.5 : 1.7, 0.5 : 1.8, 0.5 : 1.9, 0.5 : 2.0, 0.6 : 0.5, 0.6 : 0.6, 0.6 : 0.7, 0.6 : 0.8, 0.6 : 0.9, 0.6 : 1.0, 0.6 : 1.1, 0.6 : 1.2, 0.6 : 1.3, 0.6 : 1.4, 0.6 : 1.5, 0.6 : 1.6, 0.6 : 1.7, 0.6 : 1.8, 0.6 : 1.9, 0.6 : 2.0, 0.7 : 0.5, 0.7 : 0.6, 0.7 : 0.7, 0.7 : 0.8, 0.7 : 0.9, 0.7 : 1.0, 0.7 : 1.1, 0.7 : 1.2, 0.7 : 1.3, 0.7 : 1.4, 0.7 : 1.5, 0.7 : 1.6, 0.7 : 1.7, 0.7 : 1.8, 0.7 : 1.9, 0.7 : 2.0, 0.8 : 0.5, 0.8 : 0.6, 0.8 : 0.7, 0.8 : 0.8, 0.8 : 0.9, 0.8 : 1.0, 0.8 : 1.1, 0.8 : 1.2, 0.8 : 1.3, 0.8 : 1.4, 0.8 : 1.5, 0.8 : 1.6, 0.8 : 1.7, 0.8 : 1.8, 0.8 : 1.9, 0.8 : 2.0, 0.9 : 0.5, 0.9 : 0.6, 0.9 : 0.7, 0.9 : 0.8, 0.9 : 0.9, 0.9 : 1.0, 0.9 : 1.1, 0.9 : 1.2, 0.9 : 1.3, 0.9 : 1.4, 0.9 : 1.5, 0.9 : 1.6, 0.9 : 1.7, 0.9 : 1.8, 0.9 : 1.9, 0.9 : 2.0, 1.0 : 0.5, 1.0 : 0.6, 1.0 : 0.7, 1.0 : 0.8, 1.0 : 0.9, 1.0 : 1.0, 1.0 : 1.1, 1.0 : 1.2, 1.0 : 1.3, 1.0 : 1.4, 1.0 : 1.5, 1.0 : 1.6, 1.0 : 1.7, 1.0 : 1.8, 1.0 : 1.9, 1.0 : 2.0, 1.1 : 0.5, 1.1 : 0.6, 1.1 : 0.7, 1.1 : 0.8, 1.1 : 0.9, 1.1 : 1.0, 1.1 : 1.1, 1.1 : 1.2, 1.1 : 1.3, 1.1 : 1.4, 1.1 : 1.5, 1.1 : 1.6, 1.1 : 1.7, 1.1 : 1.8, 1.1 : 1.9, 1.1 : 2.0, 1.2 : 0.5, 1.2 : 0.6, 1.2 : 0.7, 1.2 : 0.8, 1.2 : 0.9, 1.2 : 1.0, 1.2 : 1.1, 1.2 : 1.2, 1.2 : 1.3, 1.2 : 1.4, 1.2 : 1.5, 1.2 : 1.6, 1.2 : 1.7, 1.2 : 1.8, 1.2 : 1.9, 1.2 : 2.0, 1.3 : 0.5, 1.3 : 0.6, 1.3 : 0.7, 1.3 : 0.8, 1.3 : 0.9, 1.3 : 1.0, 1.3 : 1.1, 1.3 : 1.2, 1.3 : 1.3, 1.3 : 1.4, 1.3 : 1.5, 1.3 : 1.6, 1.3 : 1.7, 1.3 : 1.8, 1.3 : 1.9, 1.3 : 2.0, 1.4 : 0.5, 1.4 : 0.6, 1.4 : 0.7, 1.4 : 0.8, 1.4 : 0.9, 1.4 : 1.0, 1.4 : 1.1, 1.4 : 1.2, 1.4 : 1.3, 1.4 : 1.4, 1.4 : 1.5, 1.4 : 1.6, 1.4 : 1.7, 1.4 : 1.8, 1.4 : 1.9, 1.4 : 2.0, 1.5 : 0.5, 1.5 : 0.6, 1.5 : 0.7, 1.5 : 0.8, 1.5 : 0.9, 1.5 : 1.0, 1.5 : 1.1, 1.5 : 1.2, 1.5 : 1.3, 1.5 : 1.4, 1.5 : 1.5, 1.5 : 1.6, 1.5 : 1.7, 1.5 : 1.8, 1.5 : 1.9, 1.5 : 2.0, 1.6 : 0.5, 1.6 : 0.6, 1.6 : 0.7, 1.6 : 0.8, 1.6 : 0.9, 1.6 : 1.0, 1.6 : 1.1, 1.6 : 1.2, 1.6 : 1.3, 1.6 : 1.4, 1.6 : 1.5, 1.6 1.6, 1.6 1.7, 1.6 1.8, 1.6 1.9, 1.6 2.0, 1.7 0.5, 1.7 : 0.6, 1.7 : 0.7, 1.7 : 0.8, 1.7 : 0.9, 1.7 : 1.0, 1.7 : 1.1, 1.7 : 1.2, 1.7 : 1.3, 1.7 : 1.4, 1.7 : 1.5, 1.7 : 1.6, 1.7 1.7, 1.7 1.8, 1.7 1.9, 1.7 2.0, 1.8 0.5, 1.8 0.6, 1.8 : 0.7, 1.8 : 0.8, 1.8 : 0.9, 1.8 : 1.0, 1.8 : 1.1, 1.8 : 1.2, 1.8 : 1.3, 1.8 : 1.4, 1.8 : 1.5, 1.8 : 1.6, 1.8 : 1.7, 1.8 1.8, 1.8 1.9, 1.8 2.0, 1.9 0.5, 1.9 0.6, 1.9 0.7, 1.9 : 0.8, 1.9 : 0.9, 1.9 : 1.0, 1.9 : 1.1, 1.9 : 1.2, 1.9 : 1.3, 1.9 : 1.4, 1.9 : 1.5, 1.9 : 1.6, 1.9 : 1.7, 1.9 : 1.8, 1.9 : 1.9, 1.9 : 2.0, 2.0 : 0.5, 2.0 : 0.6, 2.0 : 0.7, 2.0 : 0.8, 2.0 : 0.9, 2.0 : 1.0, 2.0 : 1.1, 2.0 : 1.2, 2.0 : 1.3, 2.0 1.4, 2.0 1.5, 2.0 1.6, 2.0 1.7, 2.0 : 1.8, 2.0 : 1.9 or 2.0 : 2.0, but is not limited thereto.

Another aspect provides a use of a composition including differentiation-induced corneal endothelial cells (CECs) and stem cells for preventing or treating a corneal disease.

Another aspect provides a method of preventing or treating a corneal disease, the method including administering a composition including an effective amount of differentiation-induced corneal endothelial cells (CECs) and stem cells to a subject in need thereof.

Another aspect provides a use of a composition including differentiation-induced corneal endothelial cells (CECs) and stem cells for the manufacture of a medicament for preventing or treating a corneal disease.

The terms, methods, and the like described for the present disclosure are identically applied among the respective aspects.

### Advantageous Effects of Invention

According to an aspect, when differentiation-induced corneal endothelial cells and stem cells of a composition are administered in combination, the composition is effective for preventing or treating corneal diseases by improving damaged corneal endothelial cells, thereby improving corneal opacity, decreasing corneal thickness, reducing intracorneal inflammation, and alleviating corneal fibrosis.

### Brief Description of Drawings

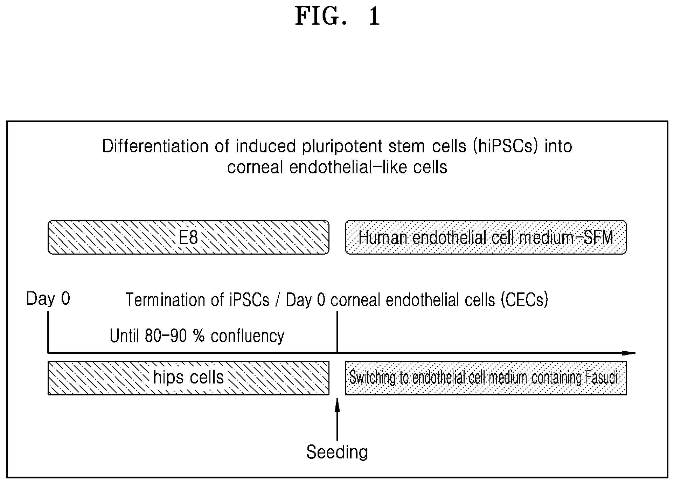

FIG. 1 is a schematic diagram illustrating a process of differentiating induced pluripotent stem cells into corneal endothelial-like cells.

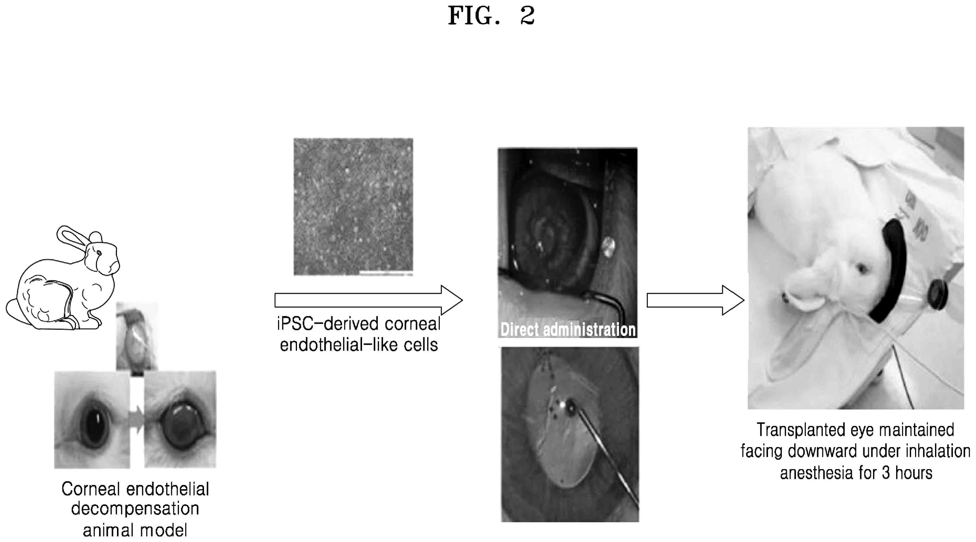

FIG. 2 is a diagram showing a transplantation process of induced corneal endothelial cells into a corneal endothelial decompensation animal model according to an aspect.

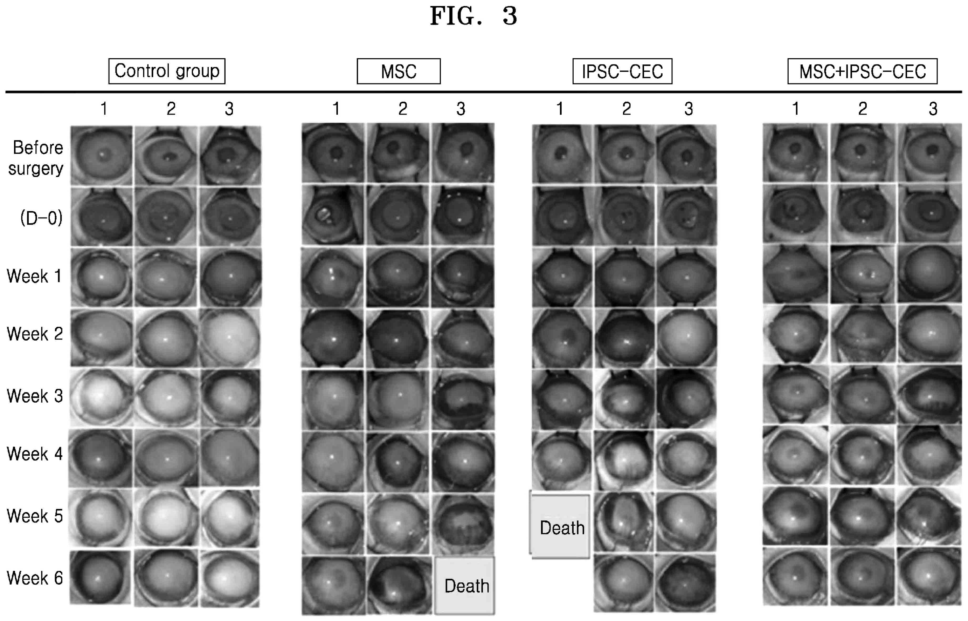

FIG. 3 presents photographs showing corneal transparency observed under a surgical microscope at 1, 2, 3, 4, 5, and 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into a corneal endothelial decompensation animal model according to an aspect.

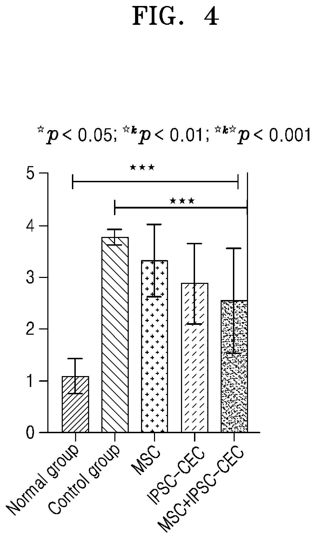

FIG. 4 is a graph illustrating results of evaluating and scoring corneal opacity for each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

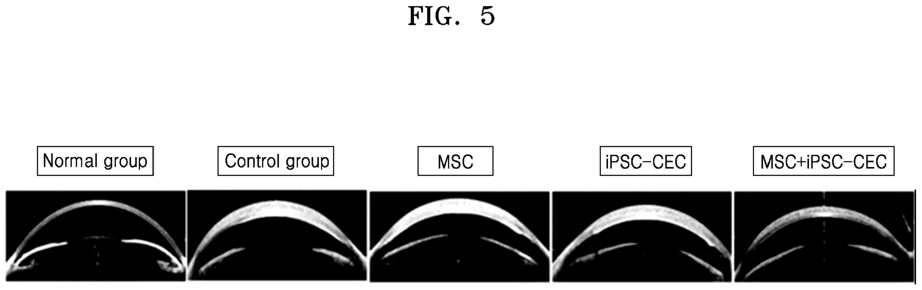

FIG. 5 is a photograph showing optical coherence tomography results for each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into an anterior chamber of a corneal endothelial decompensation animal model according to an aspect.

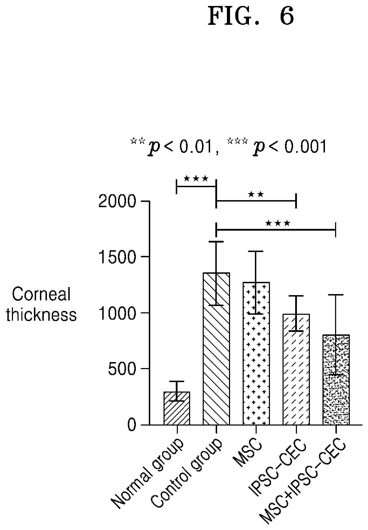

FIG. 6 is a graph illustrating results of measuring corneal thickness for each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into an anterior chamber of a corneal endothelial decompensation animal model according to an aspect.

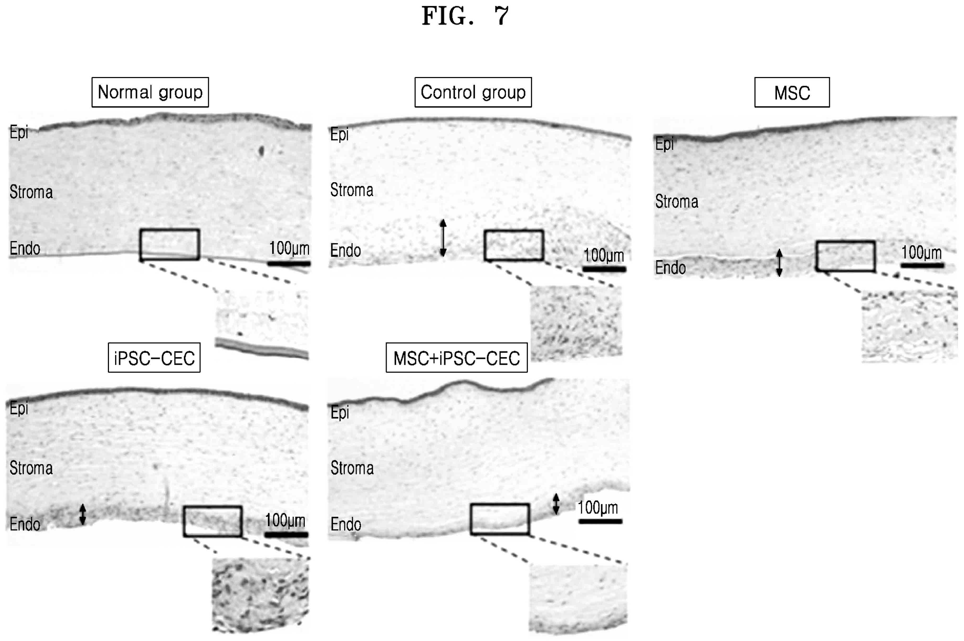

FIG. 7 presents photographs showing H&E staining results of corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of an eye of a corneal endothelial decompensation animal model according to an aspect.

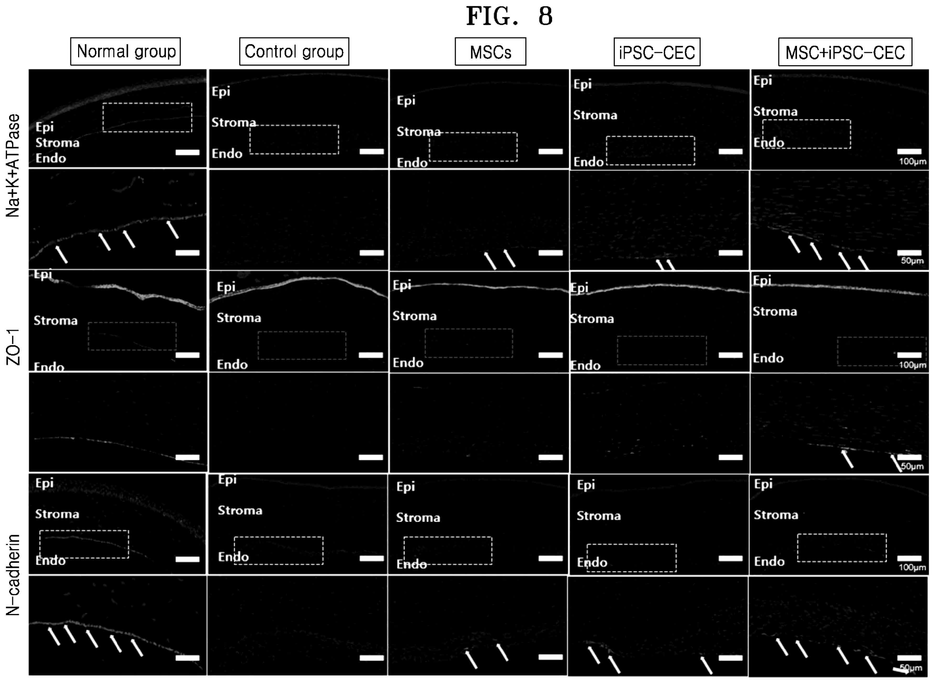

FIG. 8 is a photograph confirming expression of Na⁺/K⁺- ATPase, ZO-1, and N-cadherin in corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

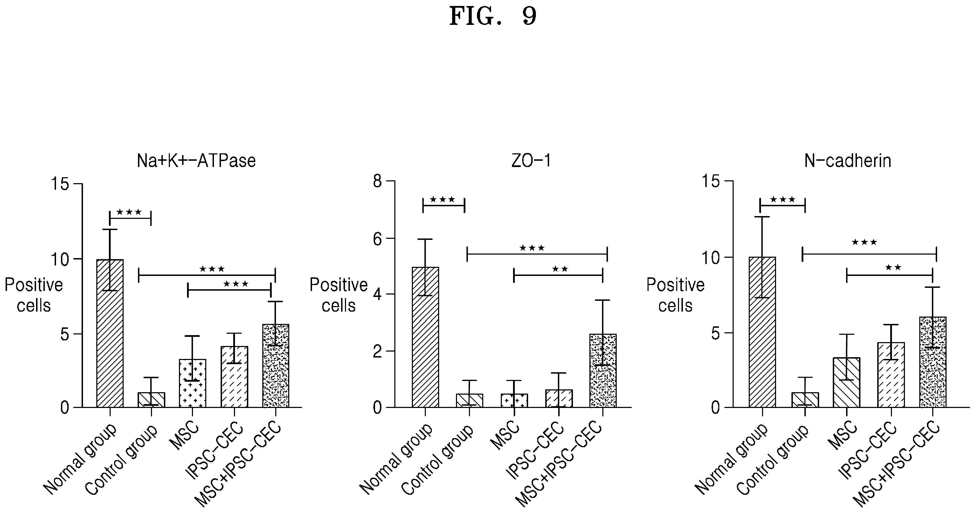

FIG. 9 is a graph showing results of confirming the number of cells expressing Na⁺/K⁺-ATPase, ZO-1, and N-cadherin in corneal tissues of experimental groups of a corneal endothelial decompensation animal model according to an aspect.

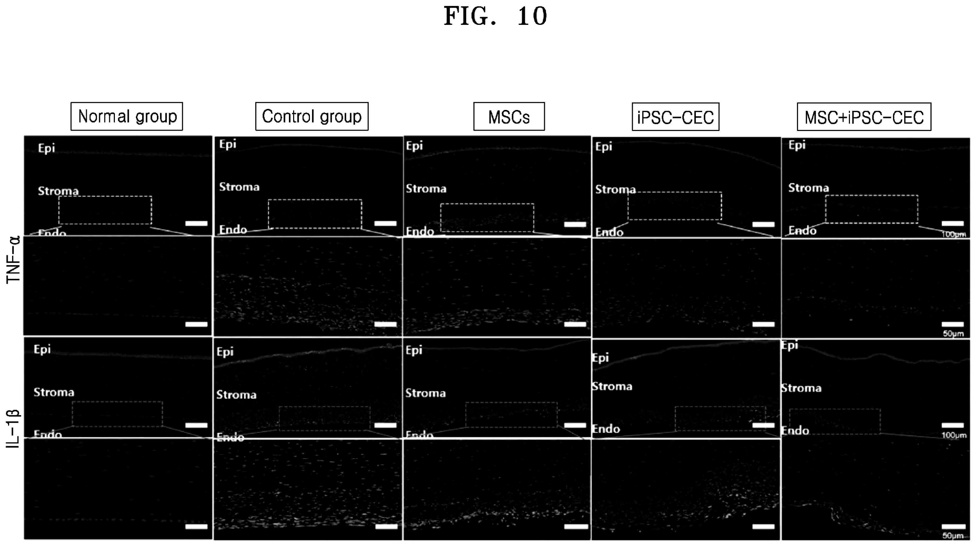

FIG. 10 shows photographs confirming the expression of TNF-α and IL-1β in corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

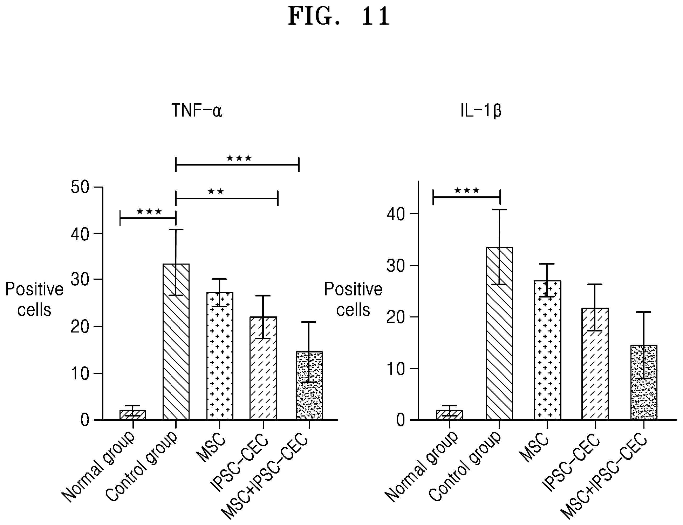

FIG. 11 is a graph illustrating the number of cells expressing TNF-α and IL-1β in the corneal tissue of a corneal endothelial decompensation animal model experimental groups according to an aspect.

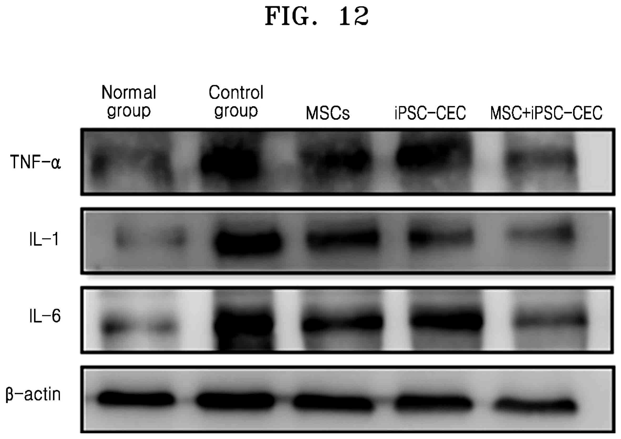

FIG. 12 presents photographs showing the results of confirming the expression of TNF-α, IL-1, and IL-6 via Western blot in corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

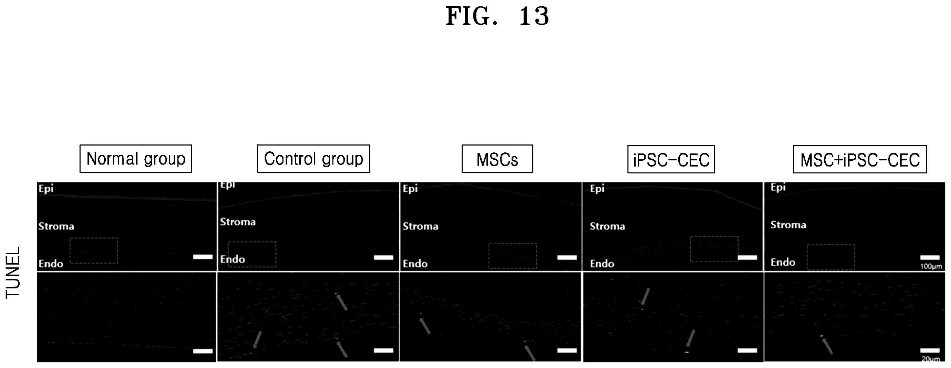

FIG. 13 shows photographs confirming apoptosis through terminal deoxynucleotidyl transferase dUTP nick end labeling (TUNEL) in corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

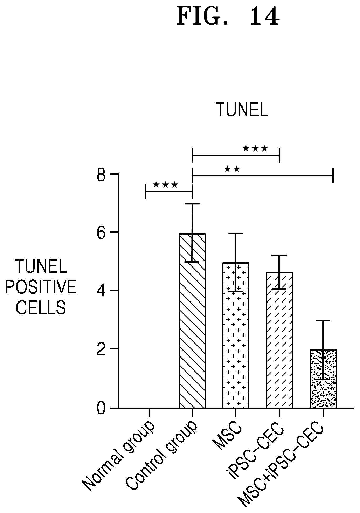

FIG. 14 is a graph illustrating the number of cells in which fragmented DNA was detected within the corneal tissue of a corneal endothelial decompensation animal model experimental groups according to an aspect.

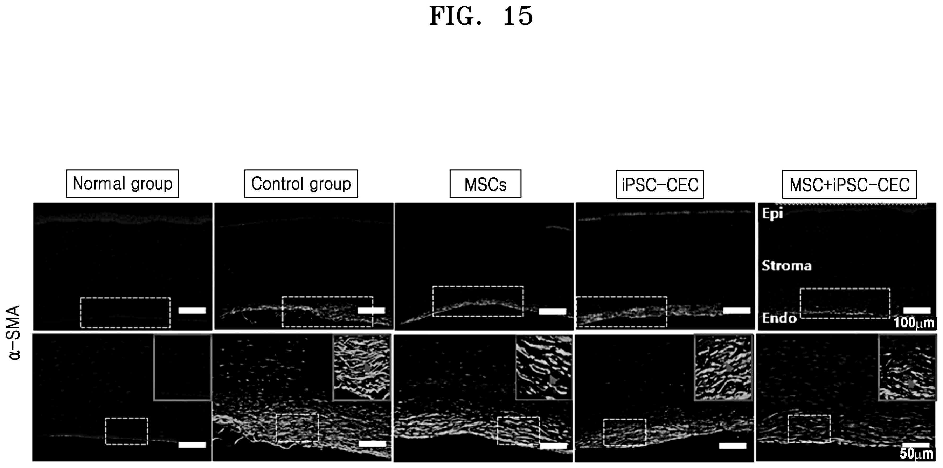

FIG. 15 shows photographs confirming the expression of α-smooth muscle actin (α-SMA) in corneas harvested from each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of a corneal endothelial decompensation animal model according to an aspect.

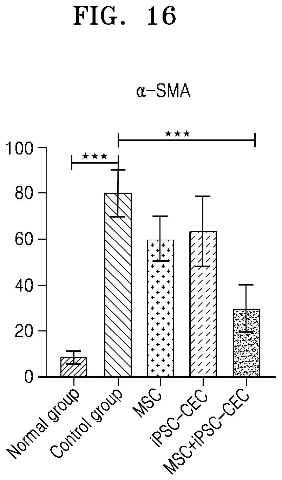

FIG. 16 is a graph illustrating the expression level of α-SMA in the corneal tissue of a corneal endothelial decompensation animal model experimental groups according to an aspect.

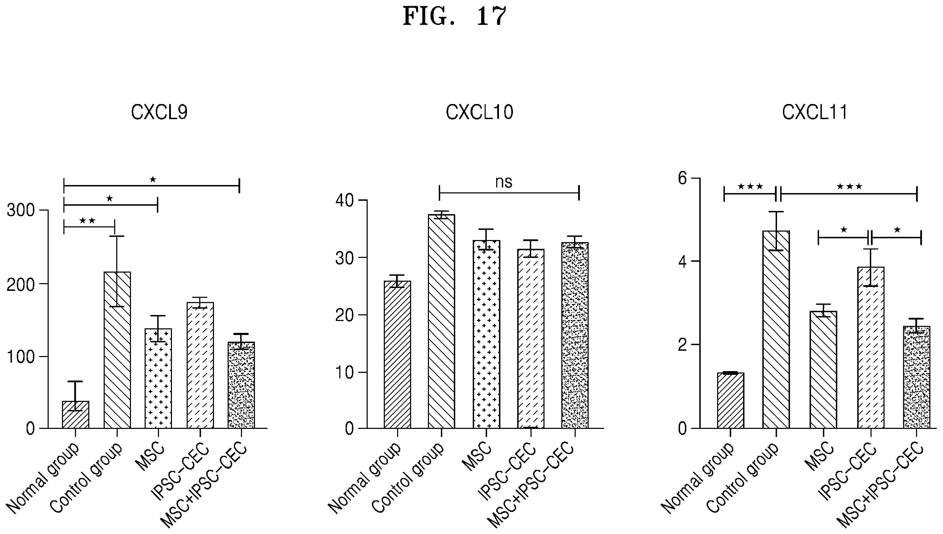

FIG. 17 is a graph illustrating the expression levels of CXCL9, CXCL10, and CXCL11 in a corneal endothelial decompensation animal model experimental groups according to an aspect.

### Mode for the Invention

Hereinafter, the present disclosure will be described in further detail with reference to examples. However, these examples are for illustrative purposes only, and the scope of the present disclosure is not limited by these examples.

### Example 1. Differentiation of Stem Cells into Corneal Endothelial Cells

Human induced pluripotent stem cells (hiPSCs) were provided by Ipsell Co., Ltd., and umbilical cord-derived mesenchymal stem cells (UC-MSCs) were provided by the Cell Therapy Center of Asan Medical Center (Asan Institute for Life Sciences, Seoul).

Human induced pluripotent stem cells (hiPSCs) or umbilical cord-derived mesenchymal stem cells (UC-MSCs) were cultured using the E8 medium (Life Technologies). Subsequently, differentiation into corneal endothelial cells was induced by replacing the medium with a medium prepared by adding supplements to Human Endothelial Serum-Free Medium (Human Endothelial-SFM). When the cells differentiated into corneal endothelial cells reached a confluence of approximately 80 to 90 %, the cells were subcultured. The differentiation process of corneal endothelial-like cells using induced pluripotent stem cells is shown in FIG. 1.

FIG. 1 is a schematic diagram illustrating the differentiation process of corneal endothelial-like cells using induced pluripotent stem cells.

Meanwhile, specific culture medium components used to induce differentiation from human induced pluripotent stem cells (hiPSCs) or umbilical cord-derived mesenchymal stem cells (UC-MSCs) into corneal endothelial cells are shown in Table 1.

**[Table 1]**

| **Cells** | **Basic Media** | **Supplements** |
|---|---|---|
| **uc-msc** | Human Endothelial SFM | Polyvinyl alcohol |
| | | SB431542 (1uM) |
| | | H-1125 (2.5uM) |
| | | 2-phosphate ascorbic acid |
| | | ITS (1%) |
| | | CaCl2 (0.2mg/mL) |
| | | Y27632 (10uM) |
| | | GSK3 inhibitor IV |
| **iPSC** | Human Endothelial SFM | SB31542 (1uM) |
| | | H-1125 (2.5uM) |
| | | 2-phosphate ascorbic acid (0.02mg/ml) |
| | | 1% ITS |
| | | hEGF (10ng/ml) |
| | | CaCl2 (0.2mg/ml) |
| | | Fasudil (10uM) |
| **Human primary CECs** | DMEM/F12 media | Fetal bovine serum (5%) |
| | | Insulin-Transferrine-Selenium (1%) |
| | | Y27632 (10uM) |
| | | Ascorbic acid (0.02mg/ml) |
| | | Cacl2 (0.2mg/ml) |
| | | Human epithelial growth factor (10ng/ml) |
| | | Antibiotic-antimycotic (1%) |

### Example 2. Confirmation of Viability, Morphological Changes, and Maintenance of Function of Induced Corneal Endothelial Cells

Stem cell-derived corneal endothelial cells, which had been differentiated by the method of Example 1 and stored in liquid nitrogen, were thawed, and the viability and morphological changes of the stem cell-derived corneal endothelial cells were analyzed. In addition, changes in the intrinsic function and intrinsic phenotype of the induced corneal endothelial cells according to subculturing were verified.

Specifically, viability of the stem cell-derived corneal endothelial-like cells was confirmed through cell counting using Trypan blue, and morphological changes of the cells were observed via microscopy. Furthermore, changes in the intrinsic function and intrinsic phenotype of the stem cell-derived corneal endothelial cells according to subculturing were verified through Western blot (WB), qPCR, and immunocytochemistry (ICC).

Through the above experiments, it was confirmed that most of the corneal endothelial cells differentiated by the method of Example 1 survived even after storage in liquid nitrogen and thawing, and the cells were observed to exhibit a hexagonal shape and tight intercellular junctions, which are representative characteristics of corneal endothelial cells. Furthermore, it was confirmed that the stem cell-derived corneal endothelial cells differentiated by the method of Example 1 maintained their intrinsic function and intrinsic phenotype even through subculturing.

### Example 3. Transplantation of Induced Corneal Endothelial Cells into a Corneal Endothelial Decompensation Animal Model

### 3.1 Preparation of Corneal Endothelial Decompensation Animal Model

Three-month-old New Zealand White rabbits were purchased from JA BIO Co., Ltd. A corneal endothelial injury animal model was prepared by removing the endothelium and stroma of the rabbit eyes using a reverse Sinskey hook.

### 3.2 Transplantation of Induced Corneal Endothelial Cells

Each cell type was transplanted into the anterior chamber of the corneal endothelial decompensation animal model, prepared by the method of Example 3.1, via direct injection.

Specifically, umbilical cord-derived mesenchymal stem cells (MSC); induced pluripotent stem cell-derived corneal endothelial cells (iPSC-CEC); or induced pluripotent stem cell-derived corneal endothelial cells and umbilical cord-derived mesenchymal stem cells (iPSC-CEC+MSC) were transplanted into the anterior chamber of the corneal endothelial decompensation animal model prepared by the method of Example 3.1.

The umbilical cord-derived mesenchymal stem cells (MSC) were transplanted at a dose of 1×10⁶ cells, the induced pluripotent stem cell-derived corneal endothelial cells (iPSC-CEC) were transplanted at a dose of 1×10⁶ cells, and the induced pluripotent stem cell-derived corneal endothelial cells and umbilical cord-derived mesenchymal stem cells (iPSC-CEC+MSC) were transplanted at a total dose of 2×10⁶ cells. In the case of co-transplanting the differentiation-induced corneal endothelial cells and umbilical cord-derived mesenchymal stem cells (iPSC-CEC+MSC), they were transplanted at a ratio of 1:1.

FIG. 2 illustrates the transplantation process of the induced corneal endothelial cells into the corneal endothelial decompensation animal model according to an aspect.

Following the transplantation of the corneal endothelial cells, the expression of human mitochondrial DNA in the corneal tissue of the corneal endothelial decompensation animal model was confirmed via PCR, and the expression of ZO-1, ATP1A1, and the like was confirmed by immunohistochemical staining. This indicates that the transplanted cells survived within the eyes of the corneal endothelial decompensation animal model and maintained the intrinsic characteristics of corneal endothelial cells.

### Experimental Example 1. Improvement of Corneal Opacity Following Transplantation of Induced Corneal Endothelial Cells

Following the transplantation of each cell type into the anterior chamber of the corneal endothelial decompensation animal model (prepared by the method of Example 3.1) according to the method of Example 3.2, corneal transparency was monitored at 1, 2, 3, 4, 5, and 6 weeks post-transplantation using a surgical microscope.

Specifically, the corneal endothelial decompensation animal model of Example 3 was divided into a control group (no cell transplantation); a group transplanted with umbilical cord-derived mesenchymal stem cells (MSC) only (Group 1: MSC); a group transplanted with induced pluripotent stem cell-derived corneal endothelial cells (iPSC-CEC) only (Group 2: iPSC-CEC); and a group co-transplanted with induced pluripotent stem cell-derived corneal endothelial cells (iPSC-CEC) and umbilical cord-derived mesenchymal stem cells (MSC) (Group 3: iPSC-CEC+MSC) to observe corneal transparency. The corneal opacity for each group was evaluated and scored from 0 to 4 (0: no corneal clouding; 1: iris details visible; 2: pupil margin visible, but iris details not visible; 3: pupil margin not discernible; and 4: total corneal opacity).

FIG. 3 presents photographs showing corneal transparency observed via a surgical microscope at 1, 2, 3, 4, 5, and 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model.

FIG. 4 is a graph illustrating the results of evaluating and scoring corneal opacity for each experimental group 6 weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of the corneal endothelial decompensation animal model (0: no corneal clouding; 1: iris details visible; 2: pupil margin visible, but iris details not visible; 3: pupil margin not discernible; and 4: total corneal opacity).

As shown in FIGS. 3 and 4, it was confirmed that the transplantation of differentiation-induced corneal endothelial cells and umbilical cord-derived mesenchymal stem cells (iPSC-CEC+MSC) significantly improved the corneal opacity of the corneal endothelial decompensation animal model compared to the control group.

This indicates that the transplantation of differentiation-induced corneal endothelial cells and umbilical cord-derived mesenchymal stem cells (iPSC-CEC+MSC) according to an embodiment of the present disclosure is effective in preventing and treating corneal endothelial decompensation.

### Experimental Example 2. Improvement of Corneal Thickness Following Transplantation of Induced Corneal Endothelial Cells

Optical coherence tomography (OCT) was performed 6 weeks post-transplantation of induced corneal endothelial cells into the anterior chamber of the corneal endothelial decompensation animal model (prepared by the method of Example 3.1) according to the method described in Example 3.2.

Specifically, the corneal endothelial decompensation animal model of Example 3 was divided into a control group (no cell transplantation), a group transplanted with umbilical cord-derived mesenchymal stem cells (MSC) only (Group 1: MSC), a group transplanted with induced pluripotent stem cell-derived corneal endothelial cells (iPSC-CEC) only (Group 2: iPSC-CEC), and a group co-transplanted with induced pluripotent stem cell-derived corneal endothelial cells (iPSC-CEC) and umbilical cord-derived mesenchymal stem cells (MSC) (Group 3: iPSC-CEC+MSC). OCT was subsequently conducted, and the results are shown in FIGS. 5 and 6.

FIG. 5 shows OCT images for each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model.

FIG. 6 is a graph illustrating the results of corneal thickness measurements for each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model.

As shown in FIGS. 5 and 6, it was confirmed that when induced pluripotent stem cell-derived corneal endothelial cells and umbilical cord-derived mesenchymal stem cells (iPSC-CEC+MSC) were co-transplanted, the corneal thickness of the corneal endothelial decompensation animal model was significantly reduced compared to the control group.

This indicates that the transplantation of induced pluripotent stem cell-derived corneal endothelial cells and umbilical cord-derived mesenchymal stem cells (iPSC-CEC+MSC) according to an embodiment of the present disclosure is effective in preventing and treating corneal endothelial decompensation.

### Experimental Example 3. Improvement of Corneal Morphology and Cell Infiltration Following Transplantation of Induced Corneal Endothelial Cells

Six weeks post-transplantation of the induced corneal endothelial cells into the anterior chamber of the corneal endothelial decompensation animal model (prepared according to the method of Example 3.1) according to the method of Example 3.2, the corneas of the corneal endothelial decompensation animal model were harvested and subjected to H&E staining to verify corneal morphology and cell infiltration, with the results shown in FIG. 7.

FIG. 7 presents photographs showing the H&E staining results of corneas harvested from each experimental group six weeks post-transplantation of umbilical cord-derived mesenchymal stem cells (MSC), differentiation-induced corneal endothelial cells (iPSC-CEC), or a combination of differentiation-induced corneal endothelial cells and umbilical cord-derived mesenchymal stem cells (iPSC-CEC+MSC) into the anterior chamber of the corneal endothelial decompensation animal model.

As shown in FIG. 7, it was confirmed that the co-transplantation of differentiation-induced corneal endothelial cells and umbilical cord-derived mesenchymal stem cells (iPSC-CEC+MSC) resulted in reduced inflammatory cell infiltration and significantly improved endothelial cell structure, exhibiting a morphology similar to a normal state, compared to other groups.

This indicates that the transplantation of differentiation-induced corneal endothelial cells and umbilical cord-derived mesenchymal stem cells (iPSC-CEC+MSC) according to an embodiment of the present disclosure is effective in preventing and treating corneal endothelial decompensation.

### Experimental Example 4. Confirmation of Improved Corneal Pump Function and Intercellular Adhesion Following Transplantation of Induced Corneal Endothelial Cells

Six weeks post-transplantation of the induced corneal endothelial cells into the anterior chamber of the corneal endothelial decompensation animal model (prepared by the method of Example 3.1) according to the method described in Example 3.2, the corneas of the corneal endothelial decompensation animal model were subjected to immunofluorescence staining and observed using a fluorescence microscope.

Specifically, corneas were collected from the control group (no cell transplantation), the group transplanted with umbilical cord-derived mesenchymal stem cells (MSC) only (Group 1: MSC), the group transplanted with induced pluripotent stem cell-derived corneal endothelial cells (iPSC-CEC) only (Group 2: iPSC-CEC), and the group co-transplanted with induced pluripotent stem cell-derived corneal endothelial cells (iPSC-CEC) and umbilical cord-derived mesenchymal stem cells (MSC) (Group 3: iPSC-CEC+MSC). The collected corneas were fixed in 4 % paraformaldehyde for 2 to 3 days, washed with PBST buffer, permeabilized in 0.5 % Triton X-100, and blocked in PBST containing 3 % bovine serum albumin (Sigma-Aldrich). The samples were incubated overnight at 4 °C with primary antibodies including anti-human ZO-1 (1:250; Invitrogen), anti-Na⁺/K⁺ ATPase (1:200; Abcam), anti-IL-1β (1:200, Santa Cruz Biotechnology), and anti-N-cadherin (1:200; Cell Signaling). Subsequently, the samples were incubated with Alexa Fluor 488-conjugated and Alexa Fluor 647-conjugated IgG secondary antibodies (1:500; Abcam, Cambridge, UK). Nuclei were counterstained with 4', 6-diamidino-2-phenylindole (DAPI; Sigma-Aldrich) for 10 minutes. Fluorescence signals were detected using a laser scanning confocal microscope (Olympus, Tokyo, Japan) or a fluorescence microscope (EVOS, Life Technologies), with the results shown in FIG. 8. Additionally, the number of cells expressing Na/⁺K⁺-ATPase, ZO-1, and N-cadherin was detected from the photographs obtained using the fluorescence microscope by using an image analysis system, with the results shown in FIG. 9.

FIG. 8 presents photographs showing the expression of Na⁺/K⁺ ATPase, ZO-1, and N-cadherin in corneas obtained from each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model, according to an aspect.

FIG. 9 is a graph illustrating the results of confirming the number of cells expressing Na⁺/K⁺-ATPase, ZO-1, and N-cadherin in the corneal tissue of the corneal endothelial decompensation animal model experimental groups according to an aspect.

As shown in FIGS. 8 and 9, it was confirmed that the number of cells expressing Na⁺/K⁺-ATPase, ZO-1, and N-cadherin significantly increased when the induced pluripotent stem cell-derived corneal endothelial cells and umbilical cord-derived mesenchymal stem cells (iPSC-CEC+MSC) were co-transplanted compared to the control group.

This indicates that corneal pump function and the formation of tight junctions were improved, demonstrating that the transplantation of induced pluripotent stem cell-derived corneal endothelial cells and umbilical cord-derived mesenchymal stem cells (iPSC-CEC+MSC) is effective for preventing or treating corneal endothelial decompensation according to an aspect of the present disclosure.

### Experimental Example 5. Confirmation of Reduction in Intracorneal Inflammation Following Transplantation of Induced Corneal Endothelial Cells

To confirm the reduction in intracorneal inflammation following the transplantation of the induced corneal endothelial cells, an experiment was performed in the same manner as in Experimental Example 4, except that an anti-human TNF-α antibody (1:250; Life Technologies) and an anti-IL-1β antibody (1:200, Santa Cruz Biotechnology) were used as primary antibodies, and the results are shown in FIGS. 10 and 11.

FIG. 10 presents photographs showing the expression of TNF-α and IL-1β in corneas harvested from each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model, according to an aspect.

FIG. 11 is a graph illustrating the number of cells expressing TNF-α and IL-1β in the corneal tissue of the corneal endothelial decompensation animal model experimental groups, according to an aspect.

Meanwhile, to confirm the effect of reducing intracorneal inflammation upon administration of umbilical cord-derived mesenchymal stem cells (MSC) and/or induced pluripotent stem cell-derived corneal endothelial cells (iPSC-CEC) to the corneal endothelial decompensation animal model, the expression of TNF-α, IL-1, and IL-6 was confirmed via Western blot.

Specifically, corneal samples collected from the rabbit model were washed in cold PBS and incubated in a lysis buffer containing phosphatase and protease inhibitors. The tissue dissociated through homogenization or sonication was centrifuged using a centrifuge to separate the supernatant, after which proteins were transferred onto a PVDF membrane via sodium dodecyl sulfate-polyacrylamide gel electrophoresis. Subsequently, blocking was performed using a TBST buffer containing bovine serum albumin. The previously prepared membrane was incubated overnight at 4 °C with IL-1, IL-6, TNF-α, and β-actin. The membrane was then washed and incubated with an HRP-linked secondary antibody. Bands were visualized using a chemiluminescence imaging system, with the results shown in FIG. 12.

FIG. 12 is a photograph illustrating the results of confirming the expression of TNF-α, IL-1, and IL-6 via Western blot in corneas harvested from each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model, according to an aspect.

As shown in FIGS. 10 to 12, it was confirmed that the number of cells expressing TNF-α, IL-1β, and IL-6 significantly decreased compared to the control group when the differentiation-induced corneal endothelial cells and umbilical cord-derived mesenchymal stem cells (iPSC-CEC+MSC) were co-transplanted.

Since TNF-α, IL-1β, and IL-6 are all cytokines associated with the promotion of inflammatory responses, the aforementioned experimental results signify that intracorneal inflammation was improved in the corneal endothelial decompensation animal model. Therefore, it demonstrates that the transplantation of induced pluripotent stem cell-derived corneal endothelial cells and umbilical cord-derived mesenchymal stem cells (iPSC-CEC+MSC) according to an aspect of the present disclosure is effective for preventing or treating corneal endothelial decompensation.

### Experimental Example 6. Confirmation of Inhibition of Intracorneal Apoptosis Following Transplantation of Induced Corneal Endothelial Cells

Six weeks post-transplantation of the induced corneal endothelial cells into the anterior chamber of the corneal endothelial decompensation animal model (prepared by the method of Example 3.1) according to the method of Example 3.2, the corneas of the corneal endothelial decompensation animal model were harvested and subjected to terminal deoxynucleotidyl transferase dUTP nick end labeling (TUNEL) staining.

Specifically, 5 µm-thick rabbit tissue sections cut from paraffin blocks were heated at 60 °C for approximately 30 minutes and subjected to dewaxing via washing with xylene and ethanol. Subsequently, the cells were stained with DAPI (4', 6-diamidino-2-phenylindole; Roche, Inc., Mannheim, Germany), and fluorescence imaging was conducted using a confocal laser scanning microscope (Carl Zeiss, Oberkochen, Germany). The distribution and number of TUNEL-positive cells were confirmed, with the results shown in FIGS. 13 and 14.

FIG. 13 presents photographs showing apoptosis confirmed through terminal deoxynucleotidyl transferase dUTP nick end labeling (TUNEL) staining in corneas harvested from each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model, according to an aspect.

FIG. 14 is a graph showing the number of cells in which fragmented DNA was detected within the corneal tissue of the corneal endothelial decompensation animal model experimental groups, according to an aspect.

As shown in FIGS. 13 and 14, it was confirmed that the number of cells in which fragmented DNA was detected significantly decreased compared to the control group when the induced pluripotent stem cell-derived corneal endothelial cells and umbilical cord-derived mesenchymal stem cells (iPSC-CEC+MSC) were co-transplanted.

This indicates that the phenomenon of intracorneal apoptosis was improved, demonstrating that the transplantation of induced pluripotent stem cell-derived corneal endothelial cells and umbilical cord-derived mesenchymal stem cells (iPSC-CEC+MSC) is effective for preventing or treating corneal endothelial decompensation according to an embodiment of the present disclosure.

### Experimental Example 7. Confirmation of Alleviation of Corneal Fibrosis Following Transplantation of Induced Corneal Endothelial Cells

To confirm the alleviation of corneal fibrosis following the transplantation of the induced corneal endothelial cells, an experiment was conducted in the same manner as in Experimental Example 4, except that an anti-human anti-α-SMA antibody (1:250; Life Technologies) was used as the primary antibody, and the results are shown in FIGS. 15 and 16.

FIG. 15 presents photographs showing the expression of α-smooth muscle actin (α-SMA) confirmed in corneas harvested from each experimental group 6 weeks post-transplantation of umbilical cord-derived mesenchymal stem cells (MSC), induced pluripotent stem cell-derived corneal endothelial cells (iPSC-CEC), or a combination of induced pluripotent stem cell-derived corneal endothelial cells and umbilical cord-derived mesenchymal stem cells (iPSC-CEC+MSC) into the anterior chamber of the corneal endothelial decompensation animal model, according to an aspect.

FIG. 16 is a graph illustrating the expression level of α-SMA in the corneal tissue of the corneal endothelial decompensation animal model experimental groups, according to an aspect.

As shown in FIGS. 15 and 16, it was confirmed that the expression level of α-smooth muscle actin (α-SMA) was significantly reduced compared to the control group when the induced pluripotent stem cell-derived corneal endothelial cells and umbilical cord-derived mesenchymal stem cells (iPSC-CEC+MSC) were transplanted according to an embodiment of the present disclosure.

This indicates that the corneal fibrosis of the experimental group of the corneal endothelial decompensation animal model was alleviated, demonstrating that the transplantation of the induced pluripotent stem cell-derived corneal endothelial cells and umbilical cord-derived mesenchymal stem cells (iPSC-CEC+MSC) is effective for preventing or treating corneal endothelial decompensation according to an embodiment of the present disclosure.

### Experimental Example 8. Confirmation of Mitigation of Allograft Rejection Reactions Following Transplantation of Induced Corneal Endothelial Cells

ELISA was performed to confirm whether allograft rejection reactions were mitigated following the transplantation of induced corneal endothelial cells and/or stem cells in a corneal endothelial decompensation animal model according to an aspect.

Specifically, reagents, standards, and samples (aqueous humor) were prepared. (CXCL-9, 10, and 11, R&D Systems). 150 µL of Assay Diluent was added to each well, and 100 µL each of the standard and control, and 75 µL of the aqueous humor were added, followed by incubation at room temperature for 2 hours. Thereafter, each well was washed four times, 200 µL of conjugate was added, and incubation was performed for 2 hours. Subsequently, each well was washed four times, 200 µL of substrate solution was added, and incubation was performed for 30 minutes while protected from light. After adding 50 µL of Stop Solution, absorbance was measured at 450 nm within 30 minutes, and the wavelength was corrected to 540 nm or 570 nm.

FIG. 17 is a graph illustrating the expression levels of CXCL9, CXCL10, and CXCL11 in the corneal endothelial decompensation animal model experimental groups according to an aspect.

As shown in FIG. 17, it was confirmed that the expression levels of CXCL9, CXCL10, and CXCL11 were reduced compared to the control group when induced pluripotent stem cell-derived corneal endothelial cells and umbilical cord-derived mesenchymal stem cells (iPSC-CEC+MSC) were co-transplanted.

This indicates that allograft rejection reactions were alleviated in the corneal endothelial decompensation animal model experimental group, demonstrating that the transplantation of induced pluripotent stem cell-derived corneal endothelial cells and umbilical cord-derived mesenchymal stem cells (iPSC-CEC+MSC) is effective for preventing or treating corneal endothelial decompensation according to an embodiment of the present disclosure.

## Claims

1. A pharmaceutical composition for preventing or treating a corneal disease, comprising differentiation-induced corneal endothelial cells (CECs) and stem cells.

2. The pharmaceutical composition for preventing or treating a corneal disease according to claim 1, wherein the composition includes the differentiation-induced CECs and the stem cells in a ratio of 0.5 to 2.0 : 0.5 to 2.0.

3. The pharmaceutical composition for preventing or treating a corneal disease according to claim 1, wherein the composition comprises the differentiation-induced CECs in an amount of 1×10⁴ to 1×10⁷ cells.

4. The pharmaceutical composition for preventing or treating a corneal disease according to claim 1, wherein the composition comprises the stem cells in an amount of 1×10⁴ cells to 1×10⁷ cells.

5. The pharmaceutical composition for preventing or treating a corneal disease according to claim 1, wherein the composition satisfies one or more of the following characteristics:
- improving corneal opacity;
- decreasing corneal thickness;
- improving corneal pump function and intercellular adhesion;
- decreasing the expression of inflammatory cytokines;
- decreasing intracorneal apoptosis;
- decreasing corneal fibrosis; and
- mitigating allograft rejection reactions.

6. The pharmaceutical composition for preventing or treating a corneal disease according to claim 1, wherein the stem cells are embryonic stem cells (ESCs), adult stem cells, or human induced pluripotent stem cells (iPSCs).

7. The pharmaceutical composition for preventing or treating a corneal disease according to claim 6, wherein the adult stem cells are mesenchymal stem cells (MSCs).

8. The pharmaceutical composition for preventing or treating a corneal disease according to claim 6, wherein the adult stem cells are selected from the group consisting of stem cells derived from bone marrow, blood, umbilical cord blood, umbilical cord, adipose tissue, liver, skin, gastrointestinal tract, placenta, and uterus.

9. The pharmaceutical composition for preventing or treating a corneal disease according to claim 1, wherein the differentiation-induced CECs are induced to differentiate from embryonic stem cells (ESCs), adult stem cells, or human induced pluripotent stem cells (iPSCs).

10. The pharmaceutical composition for preventing or treating a corneal disease according to claim 1, wherein the corneal disease is one or more selected from the group consisting of corneal endothelial decompensation, Fuchs' dystrophy, corneal endothelial cell dystrophy, corneal edema, posterior polymorphous corneal dystrophy (PPCD), and corneal transplant rejection.

11. A formulation for combined administration for preventing or treating a corneal disease, comprising:
a first agent comprising differentiation-induced corneal endothelial cells (CECs); and
a second agent comprising stem cells.

12. The formulation for combined administration for preventing or treating a corneal disease according to claim 11, wherein the first agent and the second agent are for direct ocular administration.

13. The formulation for combined administration for preventing or treating a corneal disease according to claim 11, wherein the first agent and the second agent are administered in combination simultaneously, sequentially, or in reverse order.

14. The formulation for combined administration for preventing or treating a corneal disease according to claim 11, wherein the first agent comprises the differentiation-induced corneal endothelial cells (CECs) in an amount of 1×10⁴ cells to 1×10⁷ cells.

15. The formulation for combined administration for preventing or treating a corneal disease according to claim 11, wherein the second agent comprises the stem cells in an amount of 1×10⁴ cells to 1×10⁷ cells.

16. A cell therapeutic agent for preventing or treating a corneal disease, comprising differentiation-induced corneal endothelial cells (CECs) and stem cells.

17. Use of a composition for preventing or treating a corneal disease, the composition comprising differentiation-induced corneal endothelial cells (CECs) and stem cells.

18. A method of preventing or treating a corneal disease, the method comprising administering an effective amount of a composition comprising differentiation-induced corneal endothelial cells (CECs) and stem cells, to a subject in need thereof.

19. Use of a composition comprising differentiation-induced corneal endothelial cells (CECs) and stem cells for the manufacture of a medicament for preventing or treating a corneal disease.
